# EUROPEAN PATENT SPECIFICATION

(11) **EP 0 401 503 B1**
(45) Date of publication and mention of the grant of the patent: **17.01.1996**
(21) Application number: 90107929.3
(22) Date of filing: 26.04.1990
(51) Int. Cl.: C11D 1/75, C07C 291/04

(54) **Solid non-hygroscopic trialkylamine oxides**
Feste, nicht hygroskopische Trialkylaminoxide
Trialkylaminoxides solides non-hygroscopiques

(30) Priority: 26.04.1989 US 344275; 02.10.1989 US 415910
(43) Date of publication of application: 12.12.1990
(73) Proprietor: ALBEMARLE CORPORATION, Richmond, Virginia 23219 (US)
(72) Inventor: Smith, Kim Renae, Baton Rouge, Louisiana 70810 (US); Borland, James Ellwood, Baton Rouge, Louisiana 70810 (US)
(74) Representative: Schwabe - Sandmair - Marx

(56) References cited:
- EP-A- 0 216 646
- EP-A- 0 267 662
- DE-A- 2 656 078
- DE-C- 937 058
- US-A- 4 748 275

## Description

Trialkylamine oxides are made by reacting trialkyl-amines with hydrogen peroxide. They are useful for many purposes such as in hair conditioners and shampoos as described in U. S. 3,086,943. When a C₁₀₋₁₆ alkyl dimethylamine oxide as described in U. S. 3,086,943 is made in an aqueous medium, the products will gel if the concentration of the amine oxide exceeds about 30 weight percent. Alkyl dimethylamine oxides are described in Kirk-Othmer "Encyclopedia of Chemical "Technology", 3rd ed. At page 266, Kirk-Othmer states that "when a strictly aqueous system is employed, final concentrations of amine oxide should be limited to below 35 percent since higher concentrations tend to gel and prevent good mixing."

Hoh et al., J. Am. Oil Chem. Soc., 40 (1963) pages 268-271, describe the synthesis of dimethyl dodecylamine oxide by reaction of dimethyl dodecylamine with aqueous hydrogen peroxide. With 35 weight percent hydrogen peroxide, it was necessary to add water as the reaction progressed to prevent gelling. With 70 weight percent hydrogen peroxide and an organic solvent such as ethyl acetate, a gel separated at 60 percent conversion of the amine under the conditions described by Hoh et al. causing early termination of the reaction.

DE-A-937 058 discloses that 30% H₂O₂ can be added at -40°C to a trialkylamine yielding the oxide "instantaneously". The mixture is then evaporated (or distilled) to remove water. The document deals with the preparation of low molecular weight trialkylamine oxides and their hydrates either without solvent or with very little solvent using a high percentage of H₂O₂, and carrying out the reaction at temperatures not to exceed 0°C,(right column, page 1, lines 25-32).

Chadwick U. S. 3,215,741 describes the preparation of di-C₁₋₂ alkyl C₁₀₋₂₀ alkylamine oxides by reaction of the tert-amine with hydrogen peroxide. While attempting to make the desirable concentrated solutions of the amine oxide, Chadwick found that when commercially available hydrogen peroxide containing 20-90 weight percent H₂O₂ was used, the reaction sets up to a gel resembling a thick starch paste long before completion of the reaction. Chadwick's solution to the problem was to co-feed at least 20 percent hydrogen peroxide and sufficient water to the tert-amine such that the final product was water diluted. When dimethyl dodecylamine was used, the most concentrated amine oxide solution that could be obtained was only 30-40 weight percent amine oxide.

Thus, a need exists for a solid non-hygroscopic trialkylamine oxide. Such materials would be useful as fabric softeners, in laundry detergent powders, in the preparation of detergent bars, in the preparation of shampoos and hair conditioners, in the preparation of toothpaste, in the preparation of plaque remover, in both liquid and dry laundry bleach and in any of the other uses wherein amine oxides have been used.

Stalioraitis et al. U. S. 3,776,959 describes a process for making a solution of various amine oxides in a non-polar solvent such as toluene by dissolving a tert-amine in the non-polar solvent and adding aqueous hydrogen peroxide while removing water by azeotropic distillation. The product is a solution and/or gel in an organic solvent. Such solutions could not be used in dry solid laundry detergent formulations.

In European Patent Application 0203510 there is described a process for making a concentrated aqueous gel-free solution of a di-C₆₋₁₂ alkyl methylamine oxide by reacting di-C₆₋₁₂ alkyl methylamine with aqueous hydrogen peroxide containing at least 40 weight percent H₂O₂. In this manner a gel-free aqueous solution containing 80 weight percent or more di-C₆₋₁₂ alkyl methylamine oxide can be prepared.

Smith et al. in U. S. 4,748,275 disclose that solid non-hygroscopic flakeable trialkylamine oxides can be made from di-C₁₄₋₂₄ alkyl C₁₋₂ alkylamine by reaction with aqueous hydrogen peroxide containing at least 40 weight percent H₂O₂ in the absence of a solvent and at a temperature high enough to maintain the product in a molten state. The molten product can be processed in a conventional drum flaker to produce a flaked product suitable for use in laundry detergent formulations.

Trimethylamine oxide dihydrate is known and has been used as an oxidant for organoborane compounds (Kabalka et al., J. Organometallic Chem. 125 (1977) pages 273-280).

N-methylmorpholine N-oxide monohydrate has also been reported (Maia et al., Acta Cryst. (1981) B37 pages 1858-1862). Its melting point is reported to be 348°K. It is used as a cellulose solvent for making cellulose films and fibers. It is also reported to form another hydrate melting at 39°C with 2 1/2 H₂O per mole (Chanzy et al., J. Poly. Sci., vol. 20, number 10, October 1982).

Several recent attempts to add detergent range alkyl dimethylamine oxide to granular detergent compositions such as laundry detergent have been reported. Inamorato U. S. 3,489,687 describe a process for dehydrating aqueous C₁₀₋₂₀ alkyl dimethylamine oxide solutions by adding an anhydrous salt such as sodium tripolyphosphate to the aqueous solution in an amount sufficient to consume all of the water in forming a hydrate of the added salt. This forms a soft mass of friable granules which could be compressed to form a tablet.

Stalioraitis et al. GB 1,255,102 discloses a similar process in which a salt such as sodium sulfate was added to an aqueous solution of a trialkyl amine oxide and the resulting solution is spray dried with air at 125°C to form a free-flowing granular powder containing about 58% amine oxide, 27% sodium sulfate and 9% water.

These early attempts show the need in the detergent industry for a detergent range alkyl dimethylamine oxide in a dry granular form suitable for adding to detergent composition.

It has now been discovered that discrete trialkylamine oxide dihydrates that are non-hygroscopic can be made by a process according to claim 1, i. e. by reacting a trialkylamine with concentrated aqueous hydrogen peroxide (e.g., 70 weight percent H₂O₂) in an inert organic solvent conducted such that the water/trialkyl amine mole ratio at the completion of the process is about 2/1. The trialkylamine oxide dihydrate can be recovered by crystallization from the solvent or the solvent can be removed to obtain a trialkylamine oxide dihydrate residual product.

More in detail the invention is related to a process for making a solid non-hygroscopic tert-amine oxide dihydrate, said process comprising reacting a tert-amine having the formula R¹RR³N, wherein R¹, R and R³ are independently selected from primary alkyls containing 1-30 carbon atoms, primary aralkyls containing 7-12 carbon atoms and hydroxyalkyls containing 2-4 carbon atoms and tert-amines wherein any two of R¹, R or R³ taken together form a morpholine or piperidine ring with an aqueous hydrogen peroxide at a temperature of about 20° up to the reflux temperature of the mixture in an organic using 10-90 wt% hydrogen peroxide solvent selected from organic esters, liquid aliphatic hydrocarbons containing 6-20 carbon atoms, aromatic hydrocarbons containing 6-8 carbon atoms, cycloaliphatic hydrocarbons containing 6-8 carbon atoms, aromatic halohydrocarbons containing 6-8 carbon atoms, dimethylformamide, dimethyl acetamide and mixtures thereof, said solvent being present in an amount which maintains a fluid stirrable reaction mixture and, if necessary, distilling water from or adding water to the reaction product to achieve a water/tert-amine oxide mole ratio of 1.9-2.1/1.0 and recovering said tert-amine oxide dihydrate from the reaction product.

The reactants involved in this process are tert-amines and aqueous hydrogen peroxide. The tert-amines are tri-alkyl amines in which the alkyls are straight or branched chain of the formula R¹RR³N wherein R¹, R and R³ are selected from C₁₋₃₀ primary alkyls. These include tert-amines such as trimethylamine, triethylamine, tri-n-butylamine, isobutyl dimethylamine, trihexylamine, di-n-dodecyl isobutylamine, tri-n-dodecylamine, 2-ethylhexyl dimethylamine, n-octadecyl di-n-propylamine, n-eicosyl diisobutylamine, n-triacontyl isobutyl methylamine and the like.

Other suitable tert amines include those in which one or more of the R groups are aralkyl containing 7-12 carbon atoms. Examples of these are benzyl dimethylamine, dibenzyl ethylamine, 4-tert-butylbenzyl diisobutylamine.

Likewise hydroxyalkyl substituted tert-amines can be used in the process to prepare the corresponding tert-amine oxide dihydrates. Such tert-amines include triethanolamine, dodecyl diethanolamine, didecyl ethanolamine, tetradecyl di-ethanolamine and the like.

Other tert-amines suitable for use in the process are those in which any two of R¹, R and R³ taken together form a morpholine or piperidine ring. These include N-methyl morpholine, N-ethyl morpholine, N-decyl morpholine, N-dodecyl morpholine, N-tetradecyl morpholine, N-hexadecyl morpholine, N-ethanol morpholine, N-methyl piperidine, N-ethyl piperidine, N-octyl piperidine, N-decyl piperidine, N-dodecyl piperidine, N-tetradecyl piperidine and the like.

A preferred class of tert-amines are those having the formula R¹RR³N wherein R¹ is a primary alkyl having 8-24 carbon atoms, R is a primary alkyl having either 1-2 carbon atoms or 8-24 carbon atoms and R³ is methyl or ethyl. Representative examples of these are:
n-octyl diethylamine
n-decyl dimethylamine
n-decyl diethylamine
n-dodecyl dimethylamine
n-dodecyl diethylamine
n-tetradecyl dimethylamine
n-hexadecyl diethylamine
n-octyldecyl dimethylamine
n-eicosyl dimethylamine
di-(n-octyl)methylamine
di-(n-decyl)methylamine
di-(n-dodecyl)ethylamine
di-(n-tetradecyl)methylamine
di-(n-hexadecyl)ethylamine
di-(n-octadecyl)methylamine
di-(n-eicosyl)methylamine
n-octyl n-dodecyl methylamine
n-decyl n-octadecyl ethylamine
n-decyl n-eicosyl ethylamine
and the like including mixtures thereof.

Of the above a still more preferred class of tert-amines consists of those in which R¹ is a C₈₋₂₄ primary alkyl, R is methyl or a C₈₋₂₄ primary alkyl and R₃ is methyl. Examples of these are:
octyl dimethylamine
decyl dimethylamine
dodecyl dimethylamine
tetradecyl dimethylamine
hexadecyl dimethylamine
eicosyl dimethylamine
docosyl dimethylamine
tetracosyl dimethylamine
dioctyl methylamine
didecyl methylamine
didodecyl methylamine
decyl dodecyl methylamine
ditetradecyl methylamine
tetradecyl octyl methylamine
and the like including mixtures thereof.

The second reactant, aqueous hydrogen peroxide, is preferably between 10 and 90 weight percent. The preferred embodiment uses 50-90 weight percent and more preferably 50-75 weight percent hydrogen peroxide with the most preferred embodiment being about 70 weight percent.

The amount of aqueous hydrogen peroxide should be at least about a stoichiometric amount. For example, at least about 0.9 mole and preferably 1.0 mole of hydrogen peroxide per mole of tert-amine. Good results can be achieved using 1.1-1.3 mole parts of aqueous hydrogen peroxide per mole of tert-amine and more preferably 1.15-1.25 mole parts of hydrogen peroxide per mole of tert-amine.

The reaction is conducted by adding the aqueous hydrogen peroxide to the stirred tert-amine. Inclusion of a small amount of a chelating agent such as diethylene triaminepentaacetic acid improves the reaction rate. The reaction temperature can vary from ambient up to 100°C or higher. The reaction can be started at a moderate temperature, e.g., 25-50°C and the temperature slowly increased to 60-75°C as the reaction proceeds. In most cases, little advantage will be obtained through the use of temperatures in excess of about 100°C. In fact, temperatures over 100°C may be detrimental as the amine oxide formed will decompose.

The preferred embodiment would be to add the hydrogen peroxide at a controlled rate to the amine. By controlled rate it is meant to add the hydrogen peroxide as it reacts rather than all at once so that the amount of hydrogen peroxide in the reaction mixture does not reach a hazardous level. The oxidation of the amine can be carried out at atmospheric, superatmospheric or subatmospheric pressure as may be desirable. In most cases, operation at substantially atmospheric pressure will be found to be most convenient.

Although not required, the process is preferably conducted in the presence of carbon dioxide. This serves to increase the rate of the reaction. The amount of carbon dioxide entering the liquid phase can range from 0.01 up to 5 weight percent based on the weight of the initial tert-amine. The process can even be operated under carbon dioxide pressure if desired. When using carbon dioxide the reaction can be conducted at lower temperatures, e.g., 20-40°C, with good results. As previously described, certain organic solvent must be added in an amount that maintains a fluid stirrable reaction mixture. Towards the end of reaction, for example when one-half and more preferably three-quarters of the hydrogen peroxide has been added, the reaction temperature can be allowed to increase into the 60-70°C range to complete the reaction. Of course, the entire reaction can be conducted in the higher temperature range which will decrease the amount of organic solvent required to maintain a stirrable reaction mixture. The inert organic solvents described herein can be added as needed during the hydrogen peroxide addition to maintain a stirrable reaction mixture.

Addition time will depend on temperature and scale. Preferably, the hydrogen peroxide addition is not so rapid that a large accumulation of unreacted hydrogen peroxide occurs. Addition times in the range of 0.1-8 hours for small scale runs (on the order of 1 liter) up to 1-24 hours for large scale commercial operations are generally satisfactory.

Following the hydrogen peroxide addition, the reaction mixture can be stirred at 60°C or higher for a period to be certain that the reaction has gone to completion. A ride time of 1-24 hours is usually adequate. During this time, and following hydrogen peroxide additions, organic solvent is added to the reaction as needed in order to maintain a fluid stirrable reaction mixture.

The reaction-mixture at completion will contain an amount of water that will vary with the amount and concentration of the hydrogen peroxide used in the process. For example, if 1.1 moles of 50 weight percent aqueous hydrogen peroxide is reacted with 1.0 mole of tert-amine, the reaction product will contain about 52 g (2.89 moles) of water. This exceeds the amount needed to form the dihydrate so part of this must be co-distilled out with some of the organic solvent such that the final water/amime oxide mole ratio is close to 2/1, e.g., 1.9-2,1/1.0- This can be done using a Dean-Stark water trap which returns the organic solvent. Alternatively additional organic solvent can be merely added during the distillation to prevent any gel formation and to maintain a stirrable reaction mixture.

In a more preferred embodiment the concentration of the aqueous hydrogen peroxide is about 70 weight percent. In this mode of operation, reaction of 1.1 moles of hydrogen peroxide with 1.0 mole of tert-amine will result in a reaction mixture containing 34 g (1.9 moles) of water, which is very close to the desired 2/1 water/amine oxide mole ratio. In this most preferred embodiment, it is not necessary to distill out any of the water. The tert-amine oxide dihydrate can be merely precipitated by cooling the solution. Alternatively, the organic solvent can be distilled out leaving the tert-amine oxide dihydrate as a residual product.

Good results have been achieved by conducting the process in an organic ester. This is an ester formed from an organic acid and an alcohol, aliphatic polyol or aryl-hydroxy compound.

The organic ester functions both as a polar, organic solvent for the preparation of the tert amine oxide dihydrate products of the invention and as a crystallization medium following completion of the reaction. The characteristics of the solvent are preferably such that the amine oxide dihydrate and its parent amine are both soluble at the reaction temperatures but the amine oxide dihydrate is not completely soluble at ambient temperature or lower. Thus, any organic ester which meets these criteria can be used in the practice of the present invention.

Preferred organic esters suitable for use in the invention are those having the formula: 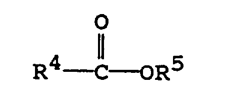 wherein R⁴ is hydrogen, (C₁₋₁₂) alkyl, (C₅₋₈) cyclo-alkyl, (C₃₋₂₄) alkenyl in which there is no unsaturation in the alkenyl group in conjugation with the carbonyl atom contained in the ester, phenyl, phenyl substituted with one or more (C₁₋₁₂) alkyl or (C₁₋₄) lower alkoxy, (C₇₋₁₂) aralkyl, benzyl substituted with one or more (C₁₋₁₂) alkyl or (C₁₋₄) lower alkoxy, (C₇₋₁₂) alkaryl, and R⁵ may be any of the organic groups represented by R⁴.

Examples of suitable alkyl groups are methyl, ethyl, n-propyl, isopropyl, n-butyl, isobutyl, sec-butyl, tert-butyl, n-amyl, and the various positional isomers thereof, and likewise the corresponding straight and branched chain isomers of hexyl, heptyl, octyl, nonyl, decyl, undecyl and dodecyl.

Examples of cycloalkyl groups are cyclopentyl, cyclohexyl and cyclooctyl.

Some examples of alkenyl groups are 1-propenyl, 1-butenyl, 2-butenyl, 3-butenyl, and the corresponding branched-chain isomers thereof as, for example, 1-isobutenyl, 2-isobutenyl, 2-sec-butenyl, including 1-methylene-2-propenyl, and the various isomers of pentenyl, hexenyl, heptenyl, octenyl, nonenyl, decenyl, undecenyl, and dodecenyl, including 3,3-dimethyl-1-butenyl, 2,3-dimethyl-1-butenyl, 2,3-dimethyl-2-butenyl, 2,3-dimethyl-3-butenyl, 1-methyl-1-ethyl-2-propenyl and the like.

Examples of (C₁₋₄) lower alkoxy groups are methoxy, ethoxy, propoxy and butoxy.

Examples of aralkyl groups are benzyl, phenylethyl, 1-phenylpropyl, 2-phenylpropyl, 3-phenylpropyl, 1- and 2-isomers of phenylisopropyl, 1-, 2-, and 3-isomers of phenylbutyl, and the like.

Examples of alkaryl groups are tolyl, 2,3-xylyl, 2,4-xylyl, 2,5-xylyl, 2,6-xylyl, 3,4-xylyl, 3,5-xylyl, o, m, and p-cumenyl, mesityl, o, m, and p-ethylphenyl.

Representative of the preferred organic esters which can be used in the practice of the invention are the methyl, ethyl, n-propyl, isopropyl, n-butyl, sec-butyl, tert-butyl, isobutyl, n-amyl, n-hexyl, 3-ethylpentyl, n-octyl, 2-ethylhexyl, cyclopentyl, cyclohexyl, 1-propenyl, 1-butenyl, 2-butenyl, 3-butenyl, 1-isobutenyl, 2-isobutenyl, 2-sec-butenyl, pentenyl, hexenyl, heptenyl, octenyl, nonenyl, decenyl, dodecenyl, phenyl, benzyl, phenylethyl, 1-phenylpropyl, 2-phenylpropyl, 3-phenylpropyl, phenylisopropyl, phenylbutyl, 2,3-xylyl, 2,4-xylyl, p-cumenyl, mesityl, m-ethylphenyl, and tolyl esters of formic, acetic, propionic, butyric, pentanoic, hexanoic, heptanoic, octanoic, benzoic, 3-methylbenzoic, 2-ethylbenzoic, 2-propylbenzoic, 2-butylbenzoic, 2-pentylbenzoic, 2-methoxybenzoic, 3-ethoxybenzoic, 4-propoxybenzoic, 5-butoxybenzoic, o-methylphenylacetic, m-ethylphenylacetic, p-propyl-phenylacetic, o-butylphenylacetic, m-hexylphenylacetic, o-octylphenylacetic, m-decylphenylacetic, p-dodecylphenyl acetic, o-methoxyphenylacetic, m-ethoxyphenylacetic, p-propoxyphenylacetic, and m-butoxyphenylacetic acids.

Carboxylic acid esters of aliphatic polyols are also useful. These esters have the formula 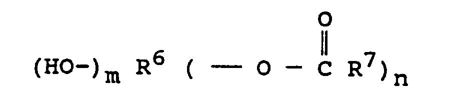 wherein R⁶ is an aliphatic hydrocarbon radical having valence m + n and containing 2-6 carbon atoms, R⁷ is hydrogen or an aliphatic hydrocarbon group containing 1-18 carbon atoms, m is zero or an integer from 1 to 3 and n is an integer from 1 to 3. Some examples of these esters are ethylene glycol diacetate, propylene glycol diacetate, butylene glycol diacetate, glycerol diformate, glycerol triacetate, glycerol monooleate, glycerol monostearate, glucose diacetate, glycerol distearate, glucose dibutyrate, glycerol tributyrate, hexane-1,6-diacetate, hexanel, 6-dilaurate, and the like.

Thus, a preferred embodiment of the present invention is a process for making a non-hygroscopic tert-amine oxide dihydrate having the formula R¹RR³NO·2H₂O wherein R¹ is a primary alkyl containing 8-24 carbon atoms, R is a primary alkyl having 1-2 carbon atoms or containing 8-24 carbon atoms and R³ is methyl or ethyl, said process comprising reacting a trialkyl amine having the formula R¹RR³N wherein R¹, R and R³ are as previously defined with at least a stoichiometric amount of aqueous hydrogen peroxide in the presence of an organic ester having the formula: 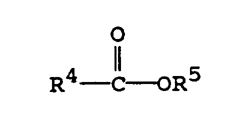 wherein R⁴ and R⁵ are as previously defined, or an ester having the formula 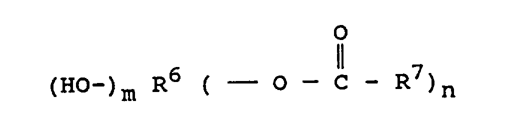 wherein R⁶, R⁷, m and n are as previously defined, said organic ester being present in an amount that prevents gel formation in the reaction mixture, distilling water from the resulting reaction mixture if necessary to adjust the water/tert-amine oxide mole ratio to about 2/1 and cooling the organic ester solvent to precipitate crystalline tert-amine dihydrate.

In a more preferred embodiment of the invention, the organic ester solvents are the C₁₋₁₂ alkyl esters of fatty acids. In a still more preferred embodiment, the organic ester is a C₁₋₅ alkyl ester of a C₂₋₅ fatty acid. Examples include methyl acetate, ethyl acetate, isopropyl acetate, n-butyl acetate, sec-butyl acetate, tert-butyl acetate, isobutyl acetate, methyl propionate, ethyl propionate, n-butyl propionate, sec-butyl propionate, isobutyl propionate, methyl butyrate, ethyl butyrate, isopropyl butyrate, ethyl valerate, amyl acetate and the like.

Thus, a more preferred embodiment of the present invention is a process for making a solid non-hygroscopic tert-amine oxide having the formula R¹RR³NO·2H₂O wherein R¹ is a primary alkyl containing 8-24 carbon atoms, R is a primary alkyl having 1-2 carbon atoms or having 8-24 carbon atoms and R³ is methyl or ethyl, said process comprising reacting a trialkyl amine having the formula R¹RR³N wherein R¹, R and R³ are as previously defined with at least a stoichiometric amount of aqueous hydrogen peroxide in the presence of a C₂₋₅ alkyl ester of a C₂₋₅ fatty acid.

The organic ester solvents of the present invention can be prepared by usual esterification procedures from a suitable alcohol and an organic acid typically in the presence of an acid catalyst (usually sulfuric acid or hydrogen chloride). For example, ethyl acetate can be prepared by simply boiling 1 mole of acetic acid with 1 mole of ethanol in the presence of sulfuric acid and recovering the ester from the reaction system. This basic principle may be modified by using an acid chloride or anhydride with an alcohol or by taking advantage of the reaction of a salt of the acid with an alkyl halide. Many of the organic esters which find use in the present invention are commercially available.

The amount of organic solvent used should be an amount sufficient to provide a readily fluid reaction mixture to avoid gelling of the reaction mass. Generally, the weight of solvent used is from less than the weight of the amine reactant to several times the weight of the amine. Usually not more than five to ten times the weight of the amine reactant of solvent need be used. In most cases, a weight of solvent of from 1 to 4 times the weight of amine reactant is most convenient.

Liquid aliphatic hydrocarbons containing 6-20 carbon atoms and cycloaliphatic hydrocarbons containing 6-8 carbon atoms can be used as the inert solvent. Of these the preferred contain 6-8 carbon atoms such as hexane, isohexane, 2-ethylhexane, heptane, octane, isooctane, cyclohexane, cyclooctane and the like.

Aromatic halohydrocarbons containing 6-8 carbon atoms and 1-2 halogen atoms can also be used as the solvent. These include chlorobenzene, dichlorobenzene, bromobenzene, chlorotoluene, 2,4-dichlorotoluene and the like.

Other useful solvents include the highly polar solvents dimethyl formamide, dimethyl acetamide, diethyl formamide, diethyl acetamide and mixtures thereof. Dimethyl sulfoxide is another useful highly polar solvent.

Mixtures of solvents can also be used with good results. For example a non-polar solvent such as toluene results in a precipitate of very fine gel-like crystals of trialkylamine oxide dihydrate. By including a small amount (e.g., 2-10 weight percent) of a polar solvent such as an alcohol, e.g., isopropanol, the crystal size can be increased making recovery by filtration much faster.

Other possible co-solvents for use with the non-polar aromatic and aliphatic hydrocarbon solvents are methanol, ethanol, isobutanol, dimethylformamide, dimethyl acetamide, dimethyl sulfoxide and the like. Other possible combinations are xylene/dimethyl formamide, benzene/ chlorobenzene, heptane/ethyl acetate, cyclohexane/isobutanol, cyclohexane/diethyl acetmaide, isooctane/dimethyl sulfoxide and the like.

Amine oxide dihydrates prepared from the parent tert-amine which have been oxidized with 65-70% hydrogen peroxide are usually soluble in the solvent, especially the organic esters, under reaction conditions and crystallize as dihydrates when cooled. Upon completion of the reaction, additional organic solvent can be added and used as a crystallization medium or the solvent may be removed by distillation or evaporation at atmospheric or reduced pressure. The molten form of the product may be readily extruded or flaked. Temperatures should be kept under about 130°C to avoid decomposition. In either case, the product is the dihydrate of the amine oxide. If a more dilute solution of hydrogen peroxide is used, e.g., 50 weight percent hydrogen peroxide, additional organic solvent can be added to the crude reaction mass which consists mainly of amine oxide, water and organic solvent upon completion of the reaction to permit removal of sufficient water via an azeotrope to obtain the amine oxide dihydrate. If aqueous solutions of hydrogen peroxide are used in which the concentration of hydrogen peroxide is in excess of approximately 70 weight percent, sufficient water must be added to the reaction mass during or after the reaction to bring the final water/tert-amine oxide mole ratio to about 2/1, e.g., 1.9-2.1/1.0. Such amounts are readily ascertainable by one skilled in the art.

Since the invention is primarily concerned with the formation of solid non-hygroscopic di-C₈₋₂₄ alkyl C₁₋₂ alkylamine oxides and solid non-hygroscopic flakeable C₈₋₂₄ alkyl di-C₁₋₂ alkylamine oxides in their dihydrate form, another embodiment of the invention is a new composition of matter consisting of trialkyl amine oxide dihydrates having the formula R¹RR³NO·2H₂O wherein R¹ is a C₈₋₂₄ primary alkyl, R is a C₁₋₂ alkyl or a C₈₋₂₄ primary alkyl and R³ is a C₁₋₂ alkyl. Of these the more preferred tert-amine oxide dihydrates are those in which R¹ is a C₈₋₂₄ primary alkyl, R is methyl or a C₈₋₂₄ primary alkyl and R³ is methyl. The most preferred tert-amine oxide dihydrates are those in which R¹ is a C₈₋₂₄ primary alkyl and R and R³ are methyl. Some examples of these are n-octyl dimethylamine oxide dihydrate, n-decyl dimethylamine oxide dihydrate, n-dodecyl dimethylamine oxide dihydrate, n-tetradecyl dimethylamine oxide dihydrate, n-hexadecyl dimethylamine oxide dihydrate and the like. The most preferred tert-amine oxide dihydrate is n-tetradecyl dimethylamine oxide dihydrate because of its superior foam properties in detergent compositions.

As mentioned previously, such tert-amine oxide dihydrates find use as fabric softeners in dry solid laundry detergent compositions.

The following examples illustrate the oxidation process according to the invention and in no manner are they intended to limit the invention described.

### Example 1

In a 250 milliliter passivated glass reaction flask was placed 100 grams of tetradecyldimethylamine (0.41 mole; amine value 230.0 mg KOH/g amine) and 0.5 gram (1.27 mmol) of diethylenetriaminepentaacetic acid. This was heated with stirring to 65°C and then 23 grams (0.47 mole) of 70 weight percent aqueous hydrogen peroxide was added dropwise over a 15-minute period. The mixture was then heated to 76°C and stirred at that temperature for 7 hours. As needed, ethyl acetate (34 mL) was added dropwise to the reaction mass in order to maintain a clear, gel-free liquid. Analysis of the crude reaction mass by proton NMR showed 99 percent amine conversion. The crude reaction mass was added to 400 mL additional ethyl acetate. The solution was then cooled to 15°C forming a non-hydroscopic white crystalline solid tetradecyldimethylamine oxide dihydrate in 86% recovered yield melting at about 41°C.

### Example 2

### (Comparative)

Example 1 was repeated except for employing only 22 grams of the hydrogen peroxide solution and adding no organic solvent to maintain a clear, gel-free liquid. After 15 minutes at 76°C the temperature was increased to 95°C in an unsuccessful attempt to fluidize the paste that had formed; and the reaction was discontinued after 24 hours. Analysis of the crude reaction mass by proton NMR showed 94% amine conversion.

### Example 3

Example 1 was repeated except that (1) the reaction temperature was 75°C instead of 76°C, (2) the solvent added dropwise as needed to maintain a clear, gel-free liquid was methyl propionate (37 mL), and (3) the crude reaction mass, after being analyzed to show 99% amine conversion, was dissolved in 300 mL of additional methyl propionate to form a solution which was cooled to 10°C to form the non-hygroscopic white crystalline solid tetradecyldimethylamine oxide dihydrate in 48% yield.

### Example 4

Example 3 was repeated except that (1) the solvent used was n-butyl acetate, (2) the amount of solvent added during the reaction was 40 mL, and (3) the temperature to which the solution of crude reaction product was cooled was 15°C. The non-hygroscopic white crystalline solid tetradecyldimethylamine oxide dihydrate was formed in 85% yield.

### Example 5

Example 4 was repeated except that (1) the solvent used was sec-butyl acetate and (2) the amount of solvent added during the reaction was 50 mL. The non-hygroscopic white crystalline solid tetradecyldimethylamine oxide dihydrate was formed in 74% yield.

### Example 6

Example 1 was repeated except that (1) the reaction was not stopped until about eight hours after completion of the peroxide addition, at which time NMR analysis showed 99+% amine conversion and (2) the reaction mass was then freed of ethyl acetate by vacuum-stripping, affording approximately 120 grams of solid tetradecyldimethylamine oxide dihydrate.

### Example 7

Example 1 was essentially repeated except that (1) the hydrogen peroxide solution was replaced with 35 grams (0.51 mole) of 50 weight percent aqueous hydrogen peroxide, (2) the amount of ethyl acetate added during the reaction was 54 mL, (3) the reaction was not stopped until about eight hours after completion of the peroxide addition, at which time NMR analysis showed 85% amine conversion, (4) the reaction mass was then diluted with an additional 65 mL of ethyl acetate, and approximately 16 mL of water was azeotroped from the mass, and (5) the remaining material was poured into 400 mL of ethyl acetate to form a solution which was held overnight in a 15°C water bath for crystallization. Filtration of the resulting mixture afforded 52 grams of crystalline tetradecyldimethylamine oxide dihydrate.

### Example 8

Example 4 was repeated except that the solvent used was methyl benzoate. The non-hygroscopic white solid tetradecyldimethylamine oxide dihydrate was formed in 79% yield.

### Example 9

In a passivated glass reaction flask was placed 100 g tetradecyldimethylamine and 0.5 g diethylenetriamine pentaacetic acid. Carbon dioxide sparge into the liquid phase was started and the mixture was stirred and heated to 65°C. The CO₂ sparge was stopped and a CO₂ gas phase was maintained over the reaction mixture. Dropwise feed of 70 weight percent aqueous hydrogen peroxide was started. At the same time, addition of ethyl acetate was commenced. After 10 minutes all the hydrogen peroxide and 28 mL of ethyl acetate had been added. Cooling was required to maintain the temperature under 75°C. Heat was applied and the reaction continued for two more hours. Dropwise addition of ethyl acetate was continued for the first 19 minutes of the two-hour period. Total ethyl acetate feed was 43 mL. The reaction mixture was a clear gel-free solution. The reaction mixture was analyzed by NMR showing a 100 percent amine conversion. The reaction mixture was poured into a flask containing 500 mL of ethyl acetate and cooled to 15°C. Needle-like crystals of tetradecyldimethylamine oxide dihydrate formed (106 g) indicating a 87 percent yield.

### Example 10

A glass reaction flask was passivated in the usual way by washing with nitric acid and rinsing with 50 weight percent aqueous hydrogen peroxide. The flask was then charged with 100 g of tetradecyldimethylamine and 0.5 g diethylenetriamine pentaacetic acid. The mixture was stirred at 65°C under a carbon dioxide atmosphere and 23 g of 70 weight percent aqueous hydrogen peroxide was added over a 5-minute period while adding N,N-dimethylacetamide as needed to maintain a fluid reaction mixture. The mixture was stirred at 75°C for two hours while adding more N,N-dimethylacetamide as needed (45 mL total) to maintain a fluid reaction mixture. The reaction mixture was poured into 200 mL N,N-dimethylacetamide and cooled to 10°C causing tetradecyl-dimethylamine oxide dihydrate to precipitate as white crystals.

### Example 11

In a passivated glass reaction flask was placed 100 g tetradecyldimethylamine and 0.5 g diethylenetriame pentaacetic acid. The mixture was stirred at 65°C under a carbon dioxide atmosphere while 23 g of 70 weight percent aqueous hydrogen peroxide was added over a 5-minute period. Stirring was continued for two hours. A total of 65 mL of toluene was added to the mixture as needed to maintain a fluid reaction. The mixture was then poured into 200 mL of toluene and cooled to 10°C causing a gel-like precipitate of tetradecyldimethylamine oxide dihydrate.

### Example 12

This experiment was carried out in the same manner as Example 11 except that 5 weight percent isopropanol was included in the toluene solvent. The final reaction mixture was also poured into toluene containing 5 weight percent isopropanol. On cooling, white crystalline tetradecyldimethylamine oxide dihydrate precipitated.

### Example 13

In a passivated glass reaction flask was placed 20 g tetradecyldimethylamine and 0.1 g diethylenetriamine pentaacetic acid. The mixture was stirred at 65°C under a carbon dioxide atmosphere while 4.6 g of 70% aqueous hydrogen peroxide was added over a 5-minute period. Stirring was continued for 2 hours at 75°C. Heptane (90 mL total) was added during the course of the reaction to maintain a fluid reaction mass. The final reaction mixture was poured into 100 mL of heptane and cooled to 10°C to give a fine crystalline precipitate of tetradecyldimethylamine oxide dihydrate which was recovered by filtration.

### Example 14

In a 250 milliliter passivated glass reaction flask was placed 100 grams of didecylmethylamine (0.32 mole; amine value 176.7 mg KOH/g amine) and 0.5 gram (1.27 mmol) of diethylenetriaminepentaacetic acid. This was heated with stirring to 65°C and then 18 grams (0.37 mole) of 70 weight percent aqueous hydrogen peroxide was added dropwise over a 15-minute period. The mixture was then heated to 76°C and stirred at that temperature for four hours. As needed, ethyl acetate (10 mL) was added dropwise to the reaction mass in order to maintain a clear, homogeneous liquid. Analysis of the crude reaction mass showed 99 percent amine conversion by proton NMR. The crude reaction mass was dissolved in 400 mL ethyl acetate. The solution was then cooled to 5°C forming a non-hygroscopic white crystalline solid didecylmethylamine oxide dihydrate in 34% yield melting at about 104°C.

The solid non-hygroscopic tert-amine oxide dihydrates made available by the present process are readily blended to form detergent compositions. Such compositions include dry powder formulations, liquid formulations and detergent bars such as toilet soap. Such compositions usually include several ingredients such as perfume, dyes, surfactants, water softeners and the like. The new tertamine oxide dihydrates are especially useful in preparing toilet soap bars. Such bars generally contain at least 25 weight percent of a compound having the formula R¹RR³NO·2H₂O wherein R¹ is a C₈₋₂₄ primary alkyl, R is methyl or a C₈₋₂₄ primary alkyl and R³ is a methyl. A typical combo bar soap will contain 5-90 weight percent of the above tert-amine oxide dihydrate, 10-95 weight percent of other surfactants, e.g., Cₗ₂₋₁₈ linear α-olefin sulfonates, sodium lauryl sulfate, sodium fatty acid soap and the like. A very useful bar soap formulation contains 25-75 weight percent C₁₀₋₁₈ alkyl dimethylamine oxide dihydrate, 0-60 weight percent sodium cocoyl isethionate, 0-20 weight percent fatty acid soap and 10-35 weight percent of a C₁₆₋₂₀ fatty acid such as stearic acid.

Very mild skin care detergent bars can be made in which the active ingredients comprise:

| Formulation | | |
|---|---|---|
| I | 20 wt % | stearic acid |
| | 80 wt % | tetradecyl dimethylamine oxide dihydrate |
| | | |
| II | 20 wt % | stearic acid |
| | 60 wt % | tetradecyl dimethylamine oxide dihydrate |
| | 20 wt % | dinonylphenol ethoxylate |
| | | |
| III | 10 wt % | coconut acid |
| | 80 wt % | cocodimethylamine oxide |
| | 10 wt % | didecyl methylamine oxide dihydrate |

Superfatted combo bars that are very mild to the skin and give a rich full lather can be made from 50-75 weight percent amine oxide dihydrate (e.g., tetradecyl dimethylamine oxide dihydrate), 25-35 weight percent fatty acid (e.g., stearic acid) and 5-20 weight percent conventional fatty acid soap. Another very useful formulation comprises 20-40 weight percent anionic surfactant (e.g., alkylbenzenesulfonates, alkyl isethionates, alkyl glycidyl sulfates, or alkyl taurates), 20-40 weight percent amine oxide dihydrate (e.g., tetradecyldimethylamine oxide dihydrate), 20-35 weight percent fatty acid (e.g., stearic acid) and 5-20 weight percent conventional fatty acid soap.

## Claims

1. A process for making a solid non-hygroscopic tert-amine oxide dihydrate, said process comprising reacting a tert-amine having the formula R¹RR³N, wherein R¹, R and R³ are independently selected from primary alkyls containing 1-30 carbon atoms, primary aralkyls containing 7-12 carbon atoms and hydroxyalkyls containing 2-4 carbon atoms and tert-amines wherein any two of R¹, R or R³ taken together form a morpholine or piperidine ring with an aqueous hydrogen peroxide said process characterized by carrying out the reaction at a temperature of 20° up to the reflux temperature of the mixture using 10-90 weight percent hydrogen peroxide in an organic solvent selected from organic esters, liquid aliphatic hydrocarbons containing 6-20 carbon atoms, cycloaliphatic hydrocarbons containing 6-8 carbon atoms, aromatic halohydrocarbons containing 6-8 carbon atoms, dimethylformamide, dimethyl acetamide and mixtures thereof, said solvent being present in an amount which maintains a fluid stirrable reaction mixture and, if necessary, distilling water from or adding water to the reaction product to achieve a water/tert-amine oxide mole ratio of 1.9-2.1/1.0 and recovering said tert-amine oxide dihydrate from the reaction product.

2. A process of Claim 1 wherein said aqueous hydrogen peroxide is a 50-90 weight percent hydrogen peroxide and said solvent is an organic ester.

3. A process of Claim 2 wherein said aqueous hydrogen peroxide is about 70 weight percent hydrogen peroxide and said organic ester is ethyl acetate.

4. A process of any of the preceding claims 1 wherein R1 is a primary alkyl containing 8-24 carbon atoms, R is a primary alkyl containing 1-2 or 8-24 carbon atoms and R³ is an alkyl containing 1-2 carbon atoms, including mixtures thereof.

5. A process of Claim 4 wherein said tert-amine has the formula R¹RR³N wherein R¹ is a primary alkyl containing 8-24 carbon atoms and R and R³ are methyl groups.

6. A process of Claim 4 wherein said tert-amine has the formula R¹RR³N wherein R¹ and R₂ are primary alkyls containing 8-24 carbon atoms and R³ is methyl.

7. A new composition of matter consisting of non-hydroscopic trialkyl amine oxide dihydrate having the formula R¹RR³NO·2H₂O wherein R¹ is a C₈₋₂₄ primary alkyl, Ris a C₁₋₂ alkyl or a C₈₋₂₄ primary alkyl and R³ is a C₁₋₂ alkyl.

8. A compound of Claim 7 which is dioctylmethylamine oxide dihydrate, didecylmethylamine oxide dihydrate, or didodecylmethylamine oxide dihydrate.

9. A compound of Claim 7 which is decyldimethyl amine oxide dihydrate, dodecyldimethylamine oxide dihydrate, tetradecyldimethylamine oxide dihydrate, hexadecyl dimethylamine oxide dihydrate, octadecyldimethylamine oxide dihydrate, or eicosyl dimethylamine oxide dihydrate.

10. A detergent composition comprising a trialkyl amine oxide dihydrate having the formula R¹RR³NO·2H₂O wherein R¹ is a C₈₋₂₄ primary alkyl, R is methyl or a C₈₋₂₄ primary alkyl and R³ is methyl.

## Patentansprüche

1. Verfahren zur Herstellung eines festen, nicht hygroskopischen tert-Aminoxiddihydrats, bei dem man ein tert-Amin der Formel R¹RR³N, in der R¹, R und R³ unabhängig voneinander aus primären Alkylen mit 1 bis 30 Kohlenstoffatomen, primären Aralkylen mit 7 bis 12 Kohlenstoffatomen und Hydroxyalkylen mit 2 bis 4 Kohlenstoffatomen ausgewählt sind, und tert-Amine, bei denen zwei beliebige aus R¹, R oder R³ zusammen genommen einen Morpholin- oder Piperidinring mit wäßrige Wasserstoffperoxid bilden, zur Umsetzung bringt,
dadurch gekennzeichnet, daß man die Reaktion bei einer Temperatur von 20°C bis zur Rückflußtemperatur der Mischung unter Verwendung von 10 bis 90 Gew.-% Wasserstoffperoxid in einem organischen Lösungsmittel, das aus organischen Estern, flüssigen aliphatischen Kohlenwasserstoffen mit 6 bis 20 Kohlenstoffatomen, cycloaliphatischen Kohlenwasserstoffen mit 6 bis 8 Kohlenstoffatomen, aromatischen Halogenkohlenwasserstoffen mit 6 bis 8 Kohlenstoffatomen, Dimethylformamid, Dimethylacetamid und deren Mischungen ausgewählt ist, durchführt, wobei das Lösungsmittel in einer solchen Menge vorhanden ist, daß eine flüssige rührbare Reaktionsmischung aufrechterhalten bleibt, und bei Bedarf Wasser aus dem Reaktionsprodukt abdestilliert oder diesem zugesetzt wird, um ein Molverhältnis von Wasser zu tert-Aminoxid von 1,9 bis 2,1 zu 1,0 zu erreichen, und das tert-Aminoxiddihydrat aus dem Reaktionsprodukt zurückgewinnt.

2. Verfahren nach Anspruch 1, bei dem das wäßrige Wasserstoffperoxid ein 50 bis 90 Gew.-%iges Wasserstoffperoxid und das Lösungsmittel ein organischer Ester ist.

3. Verfahren nach Anspruch 2, bei dem das wäßrige Wasserstoffperoxid etwa 70 Gew.-%iges Wasserstoffperoxid und der organische Ester Ethylacetat ist.

4. Verfahren nach einem der vorstehenden Ansprüche, bei dem R¹ ein primäres Alkyl mit 8 bis 24 Kohlenstoffatomen, R ein primäres Alkyl mit 1 bis 2 oder 8 bis 24 Kohlenstoffatomen und R³ ein Alkyl mit 1 bis 2 Kohlenstoffatomen ist, wobei auch Mischungen davon möglich sind.

5. Verfahren nach Anspruch 4, bei dem das tert-Amin die Formel R¹RR³N hat, in der R¹ ein primäres Alkyl mit 8 bis 24 Kohlenstoffatomen ist und R und R³ Methylgruppen sind.

6. Verfahren nach Anspruch 4, bei dem das tert-Amin die Formel R¹RR³N hat, in der R¹ und R primäre Alkylgruppen mit 8 bis 24 Kohlenstoffatomen sind und R³ Methyl ist.

7. Neue Stoffzusammensetzung, bestehend aus nicht hygroskopischem Trialkylaminoxiddihydrat der Formel R¹RR³NO·2H₂O, in der R¹ ein primäres C₈-2₄-Alkyl, R ein C₁₋₂-Alkyl oder ein primäres C₈₋₂₄-Alkyl und R³ ein C₁₋₂-Alkyl ist.

8. Verbindung nach Anspruch 7, bei der es sich um Dioctylmethylaminoxiddihydrat, Didecylmethylaminoxid-dihydrat oder Didodecylmethylaminoxiddihydrat handelt.

9. Verbindung nach Anspruch 7, bei der es sich um Decyldimethylaminoxiddihydrat, Dodecyldimethylaminoxiddihydrat, Tetradecyldimethylaminoxiddihydrat, Hexadexyldimethylaminoxiddihydrat, Octadecyldimethylaminoxiddihydrat oder Eicosyldimethylaminoxiddihydrat handelt.

10. Detergenzzusammensetzung, die ein Trialkylaminoxiddihydrat der Formel R¹RR³NO·2H₂O umfaßt, in der R¹ ein primäres C₈₋₂₄ Alkyl, R Methyl oder ein primäres C₈₋₄-Alkyl und R³ Methyl ist.

## Revendications

1. Procédé de production d'un dihydrate d'oxyde d'amine tertiaire non hygroscopique solide, ce procédé comprenant la réaction d'une amine tertiaire de formule R¹RR³N, dans laquelle R¹, R et R³ sont choisis, indépendamment, entre des groupes alkyle primaires contenant 1 à 30 atomes de carbone, des groupes aralkyle primaires contenant 7 à 12 atomes de carbone et des groupes hydroxy-alkyle contenant 2 à 4 atomes de carbone et des amines tertiaires dans lesquelles deux quelconques de R¹, R ou R³, pris ensemble, forment un noyau de morpholine ou de pipéridine, avec un peroxyde d'hydrogène aqueux, ce procédé étant caractérisé par la conduite de la réaction à une température allant de 20° jusqu'à la température de reflux du mélange avec utilisation de 10 à 90 % en poids de peroxyde d'hydrogène dans un solvant organique choisi entre des esters organiques, des hydrocarbures aliphatiques liquides contenant 6 à 20 atomes de carbone, des hydrocarbures cycloaliphatiques contenant 6 à 8 atomes de carbone, des hydrocarbures aromatiques halogénés contenant 6 à 8 atomes de carbone, le diméthylformamide, le diméthylacétamide et des mélanges de ces solvants, le solvant utilisé étant présent en une quantité qui entretient un mélange réactionnel fluide pouvant être agité et, si nécessaire, l'élimination d'eau du produit réactionnel par distillation ou l'addition d'eau au produit réactionnel pour obtenir un rapport molaire eau/oxyde d'amine tertiaire de 1,9-2,1/1,0 et l'isolement du dihydrate d'oxyde d'amine tertiaire en question du produit réactionnel.

2. Procédé suivant la revendication 1, dans lequel le peroxyde d'hydrogène aqueux est un peroxyde d'hydrogène à 50-90 % en poids et le solvant est un ester organique.

3. Procédé suivant la revendication 2, dans lequel le peroxyde d'hydrogène aqueux est du peroxyde d'hydrogène à environ 70 % en poids et l'ester organique est l'acétate d'éthyle.

4. Procédé suivant l'une quelconque des revendications 1 à 3 précédentes, dans lequel R¹ est un groupe alkyle primaire contenant 8 à 24 atomes de carbone, R est un groupe alkyle primaire contenant 1 ou 2 ou 8 à 24 atomes de carbone et R³ est un groupe alkyle contenant 1 ou 2 atomes de carbone, ainsi que des mélanges de ces radicaux.

5. Procédé suivant la revendication 4, dans lequel l'amine tertiaire répond à la formule R¹RR³N dans laquelle R¹ est un groupe alkyle primaire contenant 8 à 24 atomes de carbone et R et R³ sont des groupes méthyle.

6. Procédé suivant la revendication 4, dans lequel l'amine tertiaire répond à la formule R¹RR³N dans laquelle R¹ et R sont des groupes alkyle primaires contenant 8 à 24 atomes de carbone et R³ est un groupe méthyle.

7. Composition nouvelle, consistant en dihydrate d'oxyde de trialkylamine non hygroscopique répondant à la formule R¹RR³NO.2H₂O dans laquelle R¹ est un groupe alkyle primaire en C₈ à C₂₄, R est un groupe alkyle en C₁ ou C₂ ou un groupe alkyle primaire en C₈ à C₂₄ et R³ est un groupe alkyle en C₁ ou C₂.

8. Composé suivant la revendication 7, qui est le dihydrate d'oxyde de dioctylméthylamine, le dihydrate d'oxyde de didécylméthylamine ou le dihydrate d'oxyde de didodécyl-méthylamine.

9. Composé suivant la revendication 7, qui est le dihydrate d'oxyde de décyldiméthylamine, le dihydrate d'oxyde de dodécyldiméthylamine, le dihydrate d'oxyde de tétradécyldiméthylamine, le dihydrate d'oxyde d'hexadécyldiméthylamine, le dihydrate d'oxyde d'octadécyldiméthylamine ou le dihydrate d'oxyde d'eicosyldiméthylamine.

10. Composition détergente, comprenant un dihydrate d'oxyde de trialkylamine répondant à la formule R¹RR³NO.2H₂O dans laquelle R¹ est un groupe alkyle primaire en C₈ à C₂₄, R est un groupe méthyle ou un groupe alkyle primaire en C₈ à C₂₄ et R³ est un groupe méthyle.
